# EUROPEAN PATENT SPECIFICATION

(11) **EP 3 875 120 B1**
(45) Date of publication and mention of the grant of the patent: **13.09.2023**
(21) Application number: 20161116.7
(22) Date of filing: 05.03.2020
(51) Int. Cl.: A61L 2/04, A61L 2/20

(54) **METHOD OF STERILIZING ABSORBENT ARTICLES**
VERFAHREN ZUR STERILISIERUNG VON SAUGFÄHIGEN ARTIKELN
PROCÉDÉ DE STÉRILISATION D'ARTICLES ABSORBANTS

(43) Date of publication of application: 08.09.2021
(73) Proprietor: Ontex BV, 9255 Buggenhout (BE); Ontex Group NV, 9320 Erembodegem (BE)
(72) Inventor: Angelozzi, Pierluigi, 65123 Pescara (IT); De Lucia, Costantino, 66016 Guardiagrele Chieti (IT); Montepara, Donatella Paola, 66036 Orsogna Chieti (IT)
(74) Representative: Saurat, Thibault

(56) References cited:
- EP-A1- 3 495 553
- JP-A- 2006 272 203
- KR-B1- 101 044 439
- US-A1- 2020 001 506

## Description

### Technical field

The present invention relates to a method of sterilizing used or un-used absorbent articles by heat drying and the application of gaseous ozone under preferably dry conditions. The method includes the step of separating the absorbent articles into smaller parts, the step of heating and drying the absorbent articles to a temperature of at least 80°C and the consecutive step of directly applying ozone in gaseous state at a concentration of at least 960 ppm to the absorbent articles.

### Background

Absorbent articles such as diapers, pant-type diapers, incontinence articles, tampons, sanitary pads, panty-liners and other articles of human hygiene are widely commercially sold and used in day to day applications as well as sensitive medical applications. After use and due to the risk of contamination with human pathogens such as harmful bacteria and yeasts, most of these absorbent articles are discarded and thermally processed without recovery of the valuable raw materials. For the same reason it is common to discard un-used but faulty products from a running production process for thermal processing. A special technical problem therefore lies with the sterilization of the used or un-used absorbent articles in order to enable the recovery of the raw materials for future use.

EP3199254A1 teaches a method of recovering pulp fiber from used absorbent articles that include pulp fibers and super absorbent particles (SAP). The method includes the step of treating the used absorbent article with ozone-containing gas of about 50 g/m³ to 200 g/m³ (approx. 23350 ppm to 93400 ppm in air under standard conditions) to decompose and remove at least a portion of the SAP. The remaining materials are then decomposed into constituent elements by stirring in an aqueous solution and subsequently dried at a temperature of 100°C to 200°C. It is further taught that the ozone treatment provides the effect of a primary sterilization and that the subsequent heating and drying provides the effect of a secondary sterilization. The method has the particular disadvantage that the majority of the SAP is being gasified as CO₂ due to the high ozone concentration and thus cannot be reused in a future product or otherwise recycled. Another disadvantage comes with the safety risks of using ozone at such high concentration levels as concentrations above 3000 ppm make potential leaks in the process equipment dangerous to operating personnel. Another disadvantage lies with the requirement of drying the remaining components beyond their initial moisture content for future processing. It is also believed that the method taught provides only an insufficient sterilization effect at least due to the lack of pre-emptive drying of the used absorbent articles.

EP3495553A1 teaches a method for recovering pulp fiber from a used absorbent article. The method includes treatment of said absorbent article at a temperature of at least 80°C in an organic acid aqueous solution of a pH of no higher than 2.5 to inactivate the SAP and provide a primary sterilization effect. It is further taught that ozone may be added to the solution as an oxidizing agent at a dissolved concentration of 3 ppm to 30 ppm (approx. 1401 ppm - 14100ppm in air under standard conditions) to decompose the inactivated SAP and to carry out a secondary sterilization on the pulp fibers. It is also taught that another secondary sterilization is achieved by the subsequent heating and drying at 105°C to 210°C. The method has the particular disadvantage that the majority of the SAP is being decomposed by collapsing their three-dimensional network structure and thus making the remaining SAP unsuitable for future use. Another disadvantage lies with the requirement of drying the remaining components beyond their initial moisture content for future processing. It is also believed that the taught method provides only an insufficient sterilization effect at least due to the use of additional liquids in the treatment step. Document US2020001506 also teaches a method of producing a recycled product from structural members of used absorbent articles using an oxidizing agent treatment.

Document JP2006272203 describes a processing device that makes it possible to carbonized and recycle used paper diapers into reusable resources using ozone.

### Term definitions

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

By "substantially", it is meant at least the majority of the structure referred to.

By the terms "under dry conditions" as used herein is meant that a method step is conducted without the application of additional liquids such as water or organic solvents. It should be obvious that liquids may nevertheless be present to a certain degree due to the initial moisture content of, in particular used but also un-used, absorbent articles.

By the terms "directly applying" as used herein is meant that for example a first material or component is applied to a second material or component without any intermediary changes to said first material or component and no intermediary third component or material being present that connects said first and second material or component. In the case of direct application of ozone in gaseous state it is meant that the ozone is not solved in liquid solution as an intermediary step when applied to the used or un-used absorbent articles.

By the term "ppm" (parts per million) is meant the unit of measurement for the concentration of ozone in air under standard conditions unless otherwise provided. The following conversion rates were used herein:
- 1 g/m3 of ozone in water = 1 ppm of ozone in water
- 1 g/m3 of ozone in air = 467 ppm of ozone in air
- 1g/m3 of ozone in water = 1 g/m3 of ozone in air

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

### Summary of the invention

The present invention has been made in view of the problems mentioned above and offers a way of sufficiently sterilizing used or un-used absorbent articles from various human pathogens such as bacteria selected from the group of Escherichia Coli, Enterococcus faecalis, Staphylococcus aureus and Salmonella enterica, and pathogenic yeasts such as Candida albicans with a high yield of recoverable SAP and preferably without the need of additional subsequent drying.

This object is achieved by the method disclosed in the independent claim 1.

Embodiments of the invention are disclosed in the dependent claims.

The present invention provides a method of sterilizing one or more used or un-used absorbent articles comprising in order the steps of:
a. Providing the used or un-used absorbent articles,
b. Separating said absorbent articles into smaller parts,
c. Heating and at least partially drying said absorbent articles,
d. Directly applying ozone in gaseous state to said absorbent articles,
characterized in that the heating of step c is conducted at a temperature of at least 80° C to at most 200° C and in that the ozone in gaseous state has a concentration of at least 960 ppm to at most 3000 ppm. All steps a-d or any one or combinations of the steps a-d are conducted preferably under dry conditions. The method may be applied to at least sufficiently sterilize used or un-used absorbent articles from various human pathogens such as bacteria selected from the group of Escherichia Coli, Enterococcus faecalis, Staphylococcus aureus and Salmonella enterica, and pathogenic yeasts such as Candida albicans without substantially dissolving or changing the chemical structure of the various components, in particular SAP, of said absorbent articles. For this purpose, the used or un-used absorbent articles are advantageously separated into smaller parts in order to enable a sufficient exposure of the cellulose fluff and other components of said absorbent articles to the ozone in gaseous state. Following the separation, the absorbent articles are dried by heat to at least partially remove moisture being in particular present in used but also un-used absorbent articles. As an advantage, the application of heat provides a secondary sterilization effect. To conclude the method, ozone in gaseous state is directly applied to the used or un-used absorbent articles at relatively low concentrations to achieve the primary sterilization without substantially dissolving or changing the chemical structure of the various components, in particular the SAP, of said absorbent articles. An additional advantage of the method lies with the limited dangers to operating personals and low operating costs due to the use of low concentrations of ozone. Since preferably no additional liquids are applied to the used or un-used absorbent articles before or during the method, there are limited to no requirements of drying said absorbent articles in conclusion of the method.

Preferably, the ozone in gaseous state as applied in step d has a concentration of at least 1440 ppm to at most 2800 ppm, more preferably has a concentration of at least 1920 ppm to at most 2600 ppm and even more preferably has a concentration of about 2400 ppm. Preferably, the heating of step c is conducted for at least 5 min to at most 120 min, preferably for at least 30 min to at most 90 min, more preferably for at least 45 min to at most 75 min, even more preferably for about 60 min and/or at a temperature of at least 80° C to at most 110° C, even more preferably at least 80° C to at most 95° C, even more preferably at temperatures of at least 80° C to at most 90° C and even more preferably at temperatures of about 85° C. Surprisingly it has been found that choosing concentrations and temperatures within these preferred ranges provides the best balance between achieved sterilization and recoverable raw materials.

Preferably, step d is conducted for at least 5 min, preferably at least 15 min, more preferably at least 30 min. It has been found that the direct application of gaseous ozone for at least 5 min, preferably at least 15 min, more preferably at least 30 min, to used or un-used absorbent articles provides particularly sufficient results in respect to the sterilization effect on potentially pathogenic bacteria selected from the group of Escherichia Coli, Enterococcus faecalis, Staphylococcus aureus and Salmonella enterica, and pathogenic yeasts such as Candida albicans.

Preferably, the heating of step c comprises the steps of:
c1. pre-heating and drying the used absorbent article at a first temperature for a first amount of time,
c2. heating and drying the used absorbent article at a second temperature for a second amount of time,
wherein said first temperature is lower than said second temperature and/or said first amount of time is lower than said second amount of time. This way the heating step and drying step may be conducted within separate containers and/or with better energy efficiency thus enabling a higher and/or more efficient throughput of the overall method.

Preferably, step d follows the completion of step c in less than 40 min, more preferably follows the completion of step c2 in less than 40 min to allow for sufficient throughput of said absorbent articles during execution of the method. This way the overall execution time of the method may be reduced due to avoidance of significant delays in the transition from step c or c2 to step d.

Preferably, the completion time of step d is less than the completion time of step c, more preferably the completion time of step d is about the completion time of step c. This way the heated and dried absorbent articles may be continuously processed in step c without the waste of valuable heat energy.

Preferably, the separation of step b is conducted by an application of physical forces such as shredding and/or cutting and/or crushing and/or shaking.

Preferably, all steps are conducted at a pressure of at least 85kPa to at most 115kPa, preferably 90kPa to at most 110kPa, even more preferably of at least 95 to at most 105kPa.

Preferably, after completion of steps a-d the method comprises the additional step e of separating the smaller parts according to their chemical and/or physical structure for recycling. This may be achieved by means such as centrifugation, sieving or cyclonic separation.

Preferably, the environmental conditions of step b and/or step c are free of aqueous solution with the only moisture being present coming from the absorbent articles in their provided condition.

Preferably, the absorbent articles are used and are at least partially contaminated with urine.

Preferably, the used or un-used absorbent articles are contaminated with potentially pathogenic microbial strains selected from the group of Escherichia Coli, Enterococcus Faecalis, Staphylococcus Aureus, Salmonella Enterica and Candida Albicans.

Test examples of the method according to the disclosure will now be disclosed in order to substantiate the effectiveness. It is understood that technical features described in one or more embodiments (i.e. different ozone concentrations and heat levels) maybe combined with one or more other embodiments without departing from the intention of the disclosure and without generalization therefrom.

### Detailed description

The present invention will now be explained in more specific detail through the following test examples, with the understanding that the invention is in no way limited to the test examples.

### Materials and measuring equipment

### Test samples / absorbent articles:

- Serenity^{™} Classic (sizes large super, large maxi and ultra-large) incontinence diaper
- Serenity^{™} Soft Dry (size large) incontinence diaper

### Sterilization plant:

- Polypropylene container with lid, dimensions W 56 cm × D 36 cm × H 10 cm, equipped with a valve with a stainless-steel hose holder tap for the introduction and discharge of the Ozone;
- Ozone generator Ozonator T20 (Innovazionie Tecnologie s.r.l.);
- Humidity and temperature probe Humidity/Temp. Meter model 313A (PCE Instruments);
- Fume hood with movable arm

### Equipment and materials for laboratory analysis:

- Technical scales Talent TE 1502 (Sartorius)
- Stomacher Blender laboratory model 400
- Laminar flow biosafety hood Steril VBH MP
- Thermostat Falc ICT 521t
- Thermostat Intercontinental DAS 37000
- Thermostat Intercontinental DAS 66030
- Thermostat Memmert BE 500
- Thermostat Intercontinental DAS 37005
- 35 I Dewar container for liquid nitrogen
- Drying stove WTB Binder, FD53, Max. power consumption: 400 W
- Thermometer Testo 925 (range from -50°C to 1000°C)
- AwV C ROTRONIC - HYGROMER C94 probe
- Spray nebuliser with 20ml capacity
- Aluminium trays

### Culture media:

- LB Medium (Liofilchem), medium used for the propagation of the pure bacterial strains
- Polysorbate 80 (Tween 80) (Alfa Aesar), surfactant for the extraction of microbes from superabsorbent materials, used in 0.5% solution
- Plate Count Agar (PCA; Liofilchem), medium for total microorganism count
- Maximum Recovery Diluent (MRD; Liofilchem), diluent for the extraction of microbes from materials of various types
- TBX Agar (Liofilchem), selective medium for the Escherichia coli count
- Baird Parker Agar Base (BP, Liofilchem), selective medium for the Staphylococcal count

### Bacterial strains used for the contamination of the products:

The absorbent articles tested were contaminated with the following pure bacterial strains, stored under liquid nitrogen in a 35-litre Dewar flask, to ensure that they are maintained over time:
- Escherichia coli (ATCC 25922, Liofilchem Batch 6029510)
- Stafilococcus aureus (ATCC 25923, Liofilchem Batch 6064507)
- Enterococcus faecalis (ATCC 29212, Liofilchem Batch 5324507)
- Salmonella enterica (ATCC 14028, Liofilchem Batch 6120558)
- Candida albicans (ATCC 10231, Liofilchem Batch 6036513)

### Test methods

### Preparation of the contaminant microbial suspensions:

To prepare the microbial suspensions used for the contamination of the absorbent articles, a sterile loop was used to collect, from a vial of each bacterial strain contained in the Dewar, a quantity of glycerinate of approximately 10 µl, after a short settling time at room temperature (approximately 5 minutes). The collected aliquot was inoculated into LB broth and then incubated at 37 ± 1 °C for 24 hours (42 ± 1 °C for Salmonella enterica and 25±1 °C for Candida albicans).

The number of microorganisms present in the suspension thus prepared is determined by counting after seeding on specific selective medium (where required) of serial decimal dilutions.

### Extraction of the microorganisms:

For the extraction of microorganisms from the absorbent articles, 10 g of cellulose fluff, sterilely removed from a contaminated absorbent article, were transferred into a stomacher bag to which are added 190 ml of MRD with 0.5% Tween 80 as a non-ionic surfactant, to avoid the gelling of the superabsorbent polymer.

The bag was then extracted with a stomacher apparatus for 10 minutes.

An appropriate amount of bacterial suspension obtained as above was then seeded by inoculation in the specific selective media (1.3), selected according to the reference bacterial strain. In some tests, specified in the following, the suspension was spread plated, to improve the separation of colonies.

### Ozone flow measurement

In order to determine the quantity of ozone present in the containers used for the tests, the ozonator flow velocity was preliminarily determined by means of an acrylic variable area flowmeter, scale 0.4 -10 LPM, connected to the outlet of the instrument. The flow velocity was measured constant and equal to 7 litres/minute.

### Contamination of whole test samples:

After preparation of the initial suspension of microorganisms (2.1), 5 ml of "contaminating" liquid was seeded on each test sample or part of it and then subjected to sterilization tests. In water load tests, 500 ml of water was added after contamination of the whole absorbent article to simulate a used absorbent articles condition.

### Contamination of manually shredded absorbent articles:

After the preparation of the initial suspension of microorganisms (2.1) the absorbent articles were contaminated by diffusion with a manual action spray, in order to simulate a homogeneous and diffuse distribution of bacteria.

Initially the contamination was performed by keeping the bacterial strains separate, with an inoculum volume of 15 ml, while in subsequent tests the contamination was carried out with a suspension containing all strains.

After a suitable wait of about 10 minutes to allow for complete absorption of the liquid in the fluff, we proceeded to the manual shredding of the absorbent articles, which were then subjected to sterilization tests.

### Count of colonies of microbial strains:

Isolation and count of microorganisms were performed as required by the relevant test methods or applicable guidelines and official publications as specified below:
- Escherichia coli: UNI ISO 16449-2:2010 "Horizontal method for the count of beta-glucuronidase-positive Escherichia coli - Part 2: colony-count technique at 44°C using membranes and 5-bromo-4-chloro-3-indolyl beta-D-glucuronide".
- Staphylococcus aureus: UNI EN ISO 6888-1:2004 "Horizontal method for the count of coagulase-positive staphylococci (Staphylococcus aureus and other species) - Technique using the Baird-Parker agar medium".
- Salmonella enterica: UNI EN ISO 6579-2:2017 "Horizontal method for the detection, count and serotyping of Salmonella - Part 2: Horizontal method for the count of Salmonella spp.".
- Enterococcus faecalis: UNI EN ISO 7899-2:2003 "Research and enumeration of intestinal entero-cocci - Membrane filtration method".
- Candida albicans: UNI EN ISO 21527-2:2008 "Horizontal method for the enumeration of yeasts and moulds Colony count technique in products with water activity less than or equal to 0.95".

### Test examples

Overall, five sets of sterilization tests were conducted.

The first three test examples were focused on the study of the correlation between ozone dose and microbicide effectiveness and evaluation of the exposure time required to obtain certain levels of sterilization. The impact of the presence of water during treatment was also evaluated. The first three tests were conducted under idealized conditions, i.e., on a linear and flat surface, without any particular obstacles and with a limited volume to simulate a laminar flow.

The fourth and fifth test examples were focused on the study of the effect of heat and ozone on artificially contaminated and shredded absorbent articles, in particular to assess the effectiveness of sterilization of strains. The tests were conducted on manually shredded absorbent articles in order to simulate more realistic conditions, i.e., a turbulent flow of the ozone gas which leads to a less than optimal exposure of the contaminated areas to the ozone gas.

### Test Example 1 (ozone 2400 ppm 60 min, water/no water, cut/whole):

A set of first sterilization tests was performed to evaluate the influence of the state of the absorbent articles, whether whole or cut open, on the effectiveness of the sterilization, evaluating the bacterial inhibition in both the presence and absence of water in both conditions. Eight units of absorbent articles (Serenity^{™} Classic incontinence diaper) were contaminated with 5x109 CFU of Escherichia coli ATCC 25922 for each sample; four absorbent articles prepared in this way were examined whole, and four absorbent articles were treated after a longitudinal cut, to expose the inside of the fluff to the action of the sterilizing agent. The concentration of ozone (sterilizing agent) has always been 2400 ppm for a treatment time of 60 minutes.

It was found that, although a significant degree of elimination of bacteria is evident, being about 52% on log10, corresponding to about 4 orders of magnitude, the whole (uncut) absorbent articles does not allow complete sterilization and the presence of water substantially hinders the action of the sterilizing agent. Furthermore it was found that only for the cut absorbent articles in the absence of water a complete inhibition of the bacterial growth could be achieved.

The results obtained in the set of first sterilization tests are set out in the following Table 1:

| | **Sample type** | **Plate count** | **dilution** | **CFU** | **log10** | **% Inhibition** | **Δ log10** |
|---|---|---|---|---|---|---|---|
| Whole product | Initial suspension | 50 | 10s | 5.0 × 10₉ | 9.699 | | |
| | untreated | 45 | 10s | 4.5 **×** 10₉ | 9.653 | | |
| | untreated + water | 39 | 10s | 3.9 × 10₉ | 9.591 | | |
| | treated | 210 | 200 | 4.2 × 10₄ | 4.623 | 52.1 | -5.03 |
| | treated + water | 28 | 10s | 2.8 × 10₉ | 9.447 | 1.5 | -0.14 |
| Product cut longitudinally | untreated | 50 | 10s | 5.0 × 10₉ | 9.699 | | |
| | untreated + water | 54 | 10s | 5.4 × 10₉ | 9.732 | | |
| | treated | 0 | 0 | 0 | 0.000 | 100.0 | -9.70 |
| | treated + water | 28 | 200 | 5.6 × 10₃ | 3.748 | 61.5 | -5.98 |

### Test Example 2 (ozone 2400 ppm, 5 min, 10 min, 15 min, 30 min):

A set of second sterilization tests was performed at 2400 ppm Ozone with increased times of 5, 15 and 30 minutes in constant flow; the samples were subjected to analysis as previously performed. The tests were performed on both the ultra-large Serenity^{™} "Classic" absorbent articles, cut and dry, and on the closed ultra-large Serenity^{™} "Soft Dry" absorbent articles. The absorbent articles named "Soft Dry" are characterized by a certain degree of permeability to air, which is reflected in the inhibition of microorganisms within the fluff: unlike the Serenity^{™} "Classic" absorbent articles, which if closed do not show a significant reduction in microbial count, the "Soft Dry" absorbent articles show a good level of inhibition, although still not enough for the product to be declared sufficiently sterile. The tests were carried out on Escherichia coli as in the above.

It was found that already very short exposure times of 5 min would lead to a noticeable inhibition rates. However, complete sterilization could only be achieved at 30 min of exposure to a cut absorbent article.

The results obtained in the set of second sterilization tests are set out in the following Table 2:

| **Treatment time** | **Sample type** | **Bacterial count** | **dilution** | **CFU** | **Log10** | **% Inhibition** | **Δlog10** |
|---|---|---|---|---|---|---|---|
| | Initial suspension | 22 | 10₇ | 2.20E+08 | 8.342 | | |
| Untreated control | Portion (Serenity^{™} Classic) | 10 | 10₇ | 1.00E+08 | 8.000 | 0.0 | |
| | Whole closed (Serenity^{™} Soft Dry) | 10 | 10₇ | 1.00E+08 | 8.000 | 0.0 | 0.000 |
| Ozone 2400 ppm 5 min | Portion (Serenity^{™} Classic) | 42 | 200 | 8.40E+03 | 3.924 | 50.9 | 0.161 |
| | Whole closed (Serenity^{™} Soft Dry) | 25 | 4x10s | 1.00E+07 | 7.000 | 12.5 | 2.056 |
| Ozone 2400 ppm 15 min | Portion (Serenity^{™} Classic) | 29 | 200 | 5.80E+03 | 3.763 | 53.0 | 3.763 |
| | Whole closed (Serenity^{™} Soft Dry) | 440 | 200 | 8.80E+04 | 4.944 | 38.2 | 0.353 |
| Ozone 2400 ppm 30 min | Portion (Serenity^{™} Classic) | 0 | 200 | 0.00 | 0.000 | 100.0 | -8.000 |
| | Whole closed (Serenity^{™} Soft Dry) | 195 | 200 | 3.90E+04 | 4.591 | 42.6 | -3.409 |

### Test example 3 (ozone at different concentrations, 30min):

A set of third sterilization tests was performed to establish the EC100, i.e. the concentration at which the effectiveness of the sterilizing agent is 100%, corresponding to a total inhibition of the growth of microorganisms. To obtain growing concentrations of ozone, the intensity of the production of the sterilizing agent was reduced by adjusting the scale on the ozonator to 20%, 40%, 60%, 80% and 100%, corresponding to 480, 960, 1440, 1920 and 2400 ppm respectively. Exposure time was fixed at 30 minutes. The tests were carried out on Escherichia coli as in the above.

It was found that complete inhibition could only be obtained at the maximum concentration of the sterilizing agent. However, noticeable inhibition was already observed to start at about 960 ppm.

The results obtained in the set of third sterilization tests are set out in the following Table 3:

| **Treatment time** | **Sample type** | **Bacterial count** | **dilution** | **CFU** | **Log10** | **% Inhibition** | **Δlog10** |
|---|---|---|---|---|---|---|---|
| | Initial suspension | 25 | 10₇ | 2.50E+08 | 8.398 | | |
| Treatment 30 min. | Untreated | 18 | 10₇ | 1.80E+08 | 8.255 | 0.0 | |
| | O₃ 480 ppm | 15 | 10₇ | 1.50E+08 | 8.176 | 1.0 | -0.079 |
| | O₃ 960 ppm | 10000 | 200 | 2.00E+06 | 6.301 | 23.7 | -1.954 |
| | O₃ 1440 ppm | 98 | 200 | 1.96E+04 | 4.292 | 47.5 | -3.884 |
| | O₃ 1920 ppm | 80 | 200 | 1.60E+04 | 4.204 | 49.1 | -2.097 |
| | O₃ 2400 ppm | 0 | 200 | 0.00E+00 | 0.000 | 100.0 | -4.292 |

### Test example 4 (ozone 2400 ppm, 30min, various microbial strains):

In a set of fourth sterilization tests it was studied whether a possible shredding of the cellulose fluff could improve the propagation of the gas throughout the volume.

In a first step, the absorbent article was shredded into smaller parts of about 2-3cm diameter. The absorbent article was then in a second step contaminated by distributing 15 ml of stock solution of each strain by means of a spray nebulizer.

The use of the nebulizer allowed for a more uniform contamination compared to the pipetting method and enabled reproduction of conditions closer to real samples. After waiting 10 min for the contaminant to be absorbed by the fluff, it was shredded by hand.

On completion of the manual shredding, 10 g not subjected to sterilization to be used as positive control were collected; this sample allowed us to evaluate the correctness of the extraction procedure and to count and measure the level of contamination of the absorbent article, as well as to exclude the possibility of presence of false negatives. All the tests were carried out using the same method of contamination and preparation.

The exposure time to the adopted ozone was 30 min in continuous flushing (ozonizing machine switched on for the entire treatment time), thus keeping the process parameters established in the first stage of the study fixed.

It was found that the shredding of the cellulose fluff negatively affected the inhibition rates for at least the Escherichia coli strains as compared with the cut products of test example 1. Unfortunately, the idealized condition of test example 1 cannot be regularly achieved in an industrial environment (e.g. a laminar flow of ozone gas). However, it was also found that industrially applicable shredding would increase the inhibition rates when compared to closed/uncut product.

The results obtained in the set of fourth sterilization tests are set out in the following Table 4:

| **Strain** | **Time** | **CFU** | **Log10** | **% Inhib.** |
|---|---|---|---|---|
| Escherichia coli | 0 | 1.28E+11 | 11.107 | 0.00% |
| | 30 min | 35266.67 | 4.4633 | 59.82% |
| Enterococcus faecalis | 0 | 7.5 E+13 | 13.875 | 0.00% |
| | 30 min | 54500 | 4.736 | 65.86% |
| Staphylococcus aureus | 0 | 2.72E+12 | 12.435 | 0.00% |
| | 30 min | 300000 | 5.477 | 55.95% |
| Salmonella enterica | 0 | 6.88E+11 | 11.838 | 0.00% |
| | 30 min | 115000000 | 8.061 | 31.91% |
| Candida albicans | 0 | 66000000 | 7.820 | 0.00% |
| | 30 min | 86000 | 4.917 | 37.11% |

### Test example 5 (85°C, no ozone application):

Considering that the presence of water adversely affects the action of ozone, as amply demonstrated in the set of first sterilization tests, the effect of pre-emptive drying and heating on the microbial population inoculated into adult incontinence absorbent articles was assessed, by investigating the effect of exposure time on a potential secondary sterilization effect.

The treatment temperature of 85°C, for the verification of the minimum exposure time, was chosen on the basis of the scientific literature referring to the pasteurization procedures in the food sector: it has been demonstrated that at T > 80 °C in samples of various types of foods we observe the complete destruction of all the microorganisms present after 30 minutes of exposure. However, for the purpose of drying alone, a temperature of at least 60°C would already provide a sufficient effect.

The instrument used in this study for the heat treatment of the product is a static convection stove, brought to a temperature of 85°C; the temperature maintenance was monitored by means of a thermometer with internal heat resistant probe Pt100. The probe was inserted in a tube filled with cellulose fluff, in order more accurately to evaluate the temperature inside the product.

Initially, intervals of 5, 10, 20 and 30 minutes were conducted to evaluate the minimum exposure time to the heat necessary to achieve a secondary sterilization effect. Subsequent tests were carried out at intervals of 1, 2, 3 and 6 hours, with the addition of an overnight drying test (15pprox.. 16 hours). Only in the latter case were the tests carried out with a water load of 500 ml of saline solution (0.9% NaCl). The purpose of the last test was to verify that the time was sufficient for a complete drying of the product.

The preparation and contamination of the absorbent articles took place as already described for the ozone treatment, but using a single inoculum solution containing all the microbial strains mixed together. In detail, the solution was made up of:
- 2ml: Escherichia coli
- 2ml : Enterococcus faecalis
- 2ml : Staphylococcus aureus
- 2ml: Salmonella enterica
- 2ml: Candida albicans for a total of 10 ml.

It was found that exposure to heat would provide for a secondary sterilization effect even on bacteria and yeasts regularly not present in the food sector. The extension of the heat exposure times additionally resulted in a much higher level of growth inhibition, even for the most resistant strains.

Note that for tests in which the microbial strains were used simultaneously in a single inoculum, cross-contamination of the different strains could be observed on plates that were no longer strictly selective. In particular, in the TBX medium, specific for Escherichia coli, a massive growth of Salmonella enterica was observed, which effectively masked the actual E.coli count. Likewise, a growth of various undifferentiated microbial strains was observed in the plates of SDA used for the growth of Candida albicans for the 30 min, 1h, 2h, 3h, 6h and overnight tests. The results for Candida albicans were thus deleted from Table 8 below.

For this reason, the most selective Chromatic Coliform Agar (CCA) medium for the bacterium was chosen for the subsequent E.coli growth tests, while the SDA plates were added to with Chloramphenicol antibiotic to prevent the growth of undesirable bacteria.

The results obtained in the set of fifth sterilization tests are set out in the following Tables 5-9 for each microbial pathogen separately:

**Table 1:**

| **Treatment time** | **CFU (E. coli)** | **Log10** | **% Inhibition** | **Mean Log10** | **σ (St.dev.)** | **Mean % Inhibition** | **% σ** |
|---|---|---|---|---|---|---|---|
| 0 | 1.48E+13 | 13.170 | 0.00% | | | | |
| 5 min | 96000 | 4.982 | 62.17% | | | | |
| 10 min | 200 | 2.301 | 82.53% | | | | |
| 30 min | 2600 | 3.415 | 74.07% | 1.138 | 3.943 | 91.36% | 29.94% |
| | 0 | 0.000 | 100.00% | | | | |
| | 0 | 0.000 | 100.00% | | | | |
| 1h | 200 | 2.301 | 82.53% | 0.767 | 2.657 | 94.18% | 20.17% |
| | 0 | 0.000 | 100.00% | | | | |
| | 0 | 0.000 | 100.00% | | | | |
| 2h | 0 | 0.000 | 100.00% | 0.000 | 0.000 | 100.00% | 0.00% |
| | 0 | 0.000 | 100.00% | | | | |
| | 0 | 0.000 | 100.00% | | | | |
| 3h | 0 | 0.000 | 100.00% | 0.000 | 0.000 | 100.00% | 0.00% |
| | 0 | 0.000 | 100.00% | | | | |
| | 0 | 0.000 | 100.00% | | | | |
| 6h | 0 | 0.000 | 100.00% | | | | |
| | 0 | 0.000 | 100.00% | 0.000 | 0.000 | 100.00% | 0.00% |
| | 0 | 0.000 | 100.00% | | | | |
| overnight | 0 | 0.000 | 100.00% | | | | |

**Table 6:**

| **Treatment time** | **CFU (E. faecalis)** | **Log10** | **% Inhibition** | **Mean Log10** | **σ (St.dev.)** | **Mean % Inhibition** | **% σ** |
|---|---|---|---|---|---|---|---|
| 0 | 1.26E+14 | 14.100 | 0.00% | | | | |
| 5 min | 1.26E+14 | 14.100 | 0.00% | | | | |
| 10 min | 1.26E+14 | 14.100 | 0.00% | | | | |
| 20 min | 1.26E+14 | 14.100 | 0.00% | | | | |
| 30 min | 1.26E+14 | 14.100 | 0.00% | | | | |
| 1h | 1.26E+14 | 14.100 | 0.00% | 14.100 | 0.000 | 0.00% | 0.00% |
| | 1.26E+14 | 14.100 | 0.00% | | | | |
| | 1.26E+14 | 14.100 | 0.00% | | | | |
| 2h | 1.26E+14 | 14.100 | 0.00% | 14.100 | 0.000 | 0.00% | 0.00% |
| | 1.26E+14 | 14.100 | 0.00% | | | | |
| | 1.26E+14 | 14.100 | 0.00% | | | | |
| 3h | 238400 | 5.377 | 61.86% | 5.191 | 0.333 | 63.19% | 2.36% |
| | 137600 | 5.139 | 63.56% | | | | |
| | 114000 | 5.057 | 64.14% | | | | |
| 6h | 110800 | 5.045 | 64.22% | 5.091 | 0.264 | 63.90% | 1.87% |
| | 173600 | 5.240 | 62.84% | | | | |
| | 97200 | 4.988 | 64.63% | | | | |
| overnight | 8000 | 3.903 | 72.32% | 1.952 | 5.520 | 86.16% | 39.15% |
| | 0 | 0.000 | 100.00% | | | | |

**Table 7:**

| **Treatment time** | **CFU (S. aureus)** | **Log10** | **% Inhibition** | **Mean Log10** | **σ (St.dev.)** | **Mean % Inhibition** | **% σ** |
|---|---|---|---|---|---|---|---|
| 0 | 5.04E+12 | 12.702 | 0.00% | | | | |
| 5 min | 5.04E+12 | 12.702 | 0.00% | | | | |
| 10 min | 5.04E+12 | 12.702 | 0.00% | | | | |
| 20 min | 5.04E+12 | 12.702 | 0.00% | | | | |
| 30 min | 5.04E+12 | 12.702 | 0.00% | | | | |
| 1h | 5.04E+12 | 12.702 | 0.00% | 5.534 | 0.887 | 56.43% | 6.99% |
| | 166000 | 5.220 | 58.90% | | | | |
| | 704000 | 5.848 | 53.96% | | | | |
| 2h | 142000 | 5.152 | 59.44% | 4.570 | 1.421 | 64.03% | 11.19% |
| | 6000 | 3.778 | 70.26% | | | | |
| | 60000 | 4.778 | 62.38% | | | | |
| 3h | 8000 | 3.903 | 69.27% | 2.502 | 4.344 | 80.31% | 34.19% |
| | 0 | 0.000 | 100.00% | | | | |
| | 4000 | 3.602 | 71.64% | | | | |
| 6h | 2000 | 3.301 | 74.01% | 1.100 | 3.812 | 91.34% | 30.01% |
| | 0 | 0.000 | 100.00% | | | | |
| | 0 | 0.000 | 100.00% | | | | |
| overnight | 0 | 0.000 | 100.00% | 0.000 | | | |

**Table 8:**

| **Treatment time** | **CFU (C. albicans)** | **Log10** | **% Inhibition** | **Mean Log10** | **σ (St.dev.)** | **Mean % Inhibition** | **% σ** |
|---|---|---|---|---|---|---|---|
| 0 | 33000000 | 7.519 | 0.00% | | | | |
| 5 min | 728000 | 5.862 | 22.03% | 5.705 | 1.127 | 24.13% | 14.99% |
| | 1488000 | 6.173 | 17.90% | | | | |
| | 120000 | 5.079 | 32.44% | | | | |
| 10 min | 588000 | 5.769 | 23.26% | 5.241 | 0.998 | 30.29% | 13.27% |
| | 150000 | 5.176 | 31.16% | | | | |
| | 60000 | 4.778 | 36.45% | | | | |
| 20 min | 274000 | 5.438 | 27.68% | 5.015 | 0.785 | 33.30% | 10.44% |
| | 88000 | 4.944 | 34.24% | | | | |
| Inconclusive results for 30 min - 6h and the overnight test. | | | | | | | |

**Table 9:**

| **Treatment time** | **CFU (S. enterica)** | **Log10** | **% Inhibition** | **Mean Log10** | **σ (St.dev.)** | **Mean % Inhibition** | **%σ** |
|---|---|---|---|---|---|---|---|
| 0 | 3.77E+12 | 12.576 | 0.00% | | | | |
| 1h | 3.77E+12 | 12.576 | 0.00% | 5.290 | 0.949 | 57.94% | 7.55% |
| | 90000 | 4.954 | 60.61% | | | | |
| | 422000 | 5.625 | 55.27% | | | | |
| 2h | 3400 | 3.531 | 71.92% | 4.774 | 2.174 | 62.04% | 17.28% |
| | 174000 | 5.241 | 58.33% | | | | |
| | 354000 | 5.549 | 55.88% | | | | |
| 3h | 10000 | 4.000 | 68.19% | 4.114 | 0.395 | 67.29% | 3.14% |
| | 10000 | 4.000 | 68.19% | | | | |
| | 22000 | 4.342 | 65.47% | | | | |
| 6h | 2000 | 3.301 | 73.75% | 3.783 | 0.870 | 69.92% | 6.92% |
| | 8000 | 3.903 | 68.96% | | | | |
| | 14000 | 4.146 | 67.03% | | | | |
| overnight | 0 | 0.000 | 100.00% | 0.000 | | | |

### Summary of the test examples:

From the results of test examples 1-5 the following conclusions could be drawn:
The presence of liquids may negatively affect the sterilization effect of ozone in gaseous state when applied to used or un-used absorbent articles. Reducing the initial moisture of the absorbent articles by at least partial drying may therefore be desirable in preparation of the ozone sterilization.

Heat used for pre-emptive drying of the used or un-used absorbent articles may provide for a secondary sterilization effect at temperatures over 80°C.

The application and sterilization effect of ozone in gaseous state may be affected by the physical state of the used or un-used absorbent articles if being whole, cut open or shredded into smaller parts. It was found that inhibition of microbial pathogens might be substantially improved by shredding the absorbent articles and exposing them to a turbulent flow of ozone in gaseous state. It was further found that cutting open the absorbent articles and exposing them to a laminar flow of ozone might improve the inhibition even further. However, it was also concluded that shredding (separating the absorbent article into smaller parts) was preferred as a suitable solution for industrial application.

The concentration of ozone in gaseous state during application may affect the sterilizing effect. Noticeable inhibition rates may first be observed at 960 ppm.

## Claims

1. A method of sterilizing one or more used or un-used absorbent articles comprising in order the steps of:
a. Providing the used or un-used absorbent articles,
b. Separating said absorbent articles into smaller parts,
c. Heating and at least partially drying said absorbent articles,
d. Directly applying ozone in gaseous state to said absorbent articles,
**characterized in that** the heating of step c is conducted at a temperature of at least 80° C to at most 200° C and **in that** the ozone in gaseous state has a concentration of at least 960 ppm or 2.05567 g/m³ in air to at most 3000 ppm or 6.42398 g/m³ in air.

2. A method according to claim 1, **characterized in that** the ozone in gaseous state has a concentration of at least 1440 ppm or 3.08351 g/m³ in air to at most 2800 ppm or 5.99572 g/m³ in air, preferably has a concentration of at least 1920 ppm or 4.11135 g/m³ in air to at most 2600 ppm or 5.56745 g/m³ in air, even more preferably has a concentration of about 2400 ppm or 5.13919 g/m³ in air.

3. A method according to any one of the preceding claims, **characterized in that** step c is conducted for at least 5 min to at most 120 min, preferably for at least 30 min to at most 90 min, more preferably for at least 45 min to at most 75 min, even more preferably for about 60 min.

4. A method according to any one of the preceding claims, **characterized in that** the heating of step c is conducted at a temperature of at least 80° C to at most 110° C, preferably at least 80° C to at most 95° C, even more preferably at a temperature of at least 80° C to at most 90° C, even more preferably at a temperature of about 85° C.

5. A method according to any one of the preceding claims, **characterized in that** step d is conducted for at least 5 min, preferably at least 15 min, even more preferably at least 30 min.

6. A method according to any one of the preceding claims, **characterized in that** step c comprises the steps of:
c1. pre-heating and drying the used absorbent article at a first temperature for a first amount of time,
c2. heating and drying the used absorbent article at a second temperature for a second amount of time,
wherein said first temperature is lower than said second temperature and/or said first amount of time is lower than said second amount of time.

7. A method according to any one of the preceding claims, **characterized in that** step d follows the completion of step c in less than 40 min.

8. A method according to any one of the preceding claims, **characterized in that** the completion time of step d is less than the completion time of step c, more preferably the completion time of step d is about the completion time of step c.

9. A method according to any one of the preceding claims, **characterized in that** step b is conducted by an application of physical forces such as shredding and/or cutting and/or crushing and/or shaking.

10. A method according to any one of the preceding claims, **characterized in that** all steps are conducted at a pressure of at least 85kPa to at most 115kPa, preferably 90kPa to at most 110kPa, even more preferably of at least 95kPa to at most 105kPa.

11. A method according to any one of the preceding claims, **characterized in that** after completion of steps a-d the method comprises the additional step e of separating the smaller parts according to their chemical and/or physical structure for recycling.

12. A method according to any one of the preceding claims, **characterized in that** the environmental conditions of step b and/or step c are free of aqueous solution.

13. A method according to any one of the preceding claims, **characterized in that** the absorbent articles are used and at least partially contaminated with urine.

14. A method according to any one of the preceding claims, **characterized in that** the used or un-used absorbent articles are contaminated with potentially pathogenic microbial strains selected from the group of Escherichia Coli, Enterococcus Faecalis, Staphylococcus Aureus, Salmonella Enterica and Candida Albicans.

## Patentansprüche

1. Verfahren zur Sterilisierung von einem oder mehreren benutzten oder unbenutzten saugfähigen Artikeln, umfassend die folgenden Schritte:
a. Bereitstellen des benutzten oder unbenutzten saugfähigen Artikels,
b. Trennen der saugfähigen Artikel in kleinere Teile,
c. Erhitzen und mindestens teilweises Trocken der saugfähigen Artikel,
d. direktes Anwenden von Ozon in gasförmigem Zustand auf die saugfähigen Artikel, **dadurch gekennzeichnet, dass** das Erhitzen von Schritt c bei einer Temperatur von mindestens 80 °C bis höchstens 200 °C durchgeführt wird, und dadurch, dass das Ozon in gasförmigem Zustand eine Konzentration von mindestens 960 ppm oder 2,05567 g/m³ in Luft bis höchstens 3000 ppm oder 6,42398 g/m³ in Luft aufweist.

2. Verfahren nach Anspruch 1, **dadurch gekennzeichnet, dass** das Ozon in gasförmigem Zustand eine Konzentration von mindestens 1440 ppm oder 3,08351 g/m³ in Luft bis höchstens 2800 ppm oder 5,99572 g/m³ in Luft aufweist, vorzugsweise eine Konzentration von mindestens 1920 ppm oder 4,11135 g/m³ in Luft bis höchstens 2600 ppm oder 5,56745 g/m³ in Luft aufweist, noch bevorzugter eine Konzentration von ungefähr 2400 ppm oder 5,13919 g/m3 in Luft aufweist.

3. Verfahren nach einem der vorhergehenden Ansprüche, **dadurch gekennzeichnet, dass** Schritt c während mindestens 5 min bis höchstens 120 min durchgeführt wird, vorzugsweise während mindestens 30 min bis höchstens 90 min, insbesondere während mindestens 45 min bis höchstens 75 min, noch bevorzugter während ungefähr 60 min.

4. Verfahren nach einem der vorhergehenden Ansprüche, **dadurch gekennzeichnet, dass** das Erhitzen von Schritt c bei einer Temperatur von mindestens 80 °C bis höchstens 110 °C durchgeführt wird, vorzugsweise mindestens 80 °C bis höchstens 95 °C, noch bevorzugter bei einer Temperatur von mindestens 80 °C bis höchstens 90 °C, noch bevorzugter bei einer Temperatur von ungefähr 85 °C.

5. Verfahren nach einem der vorhergehenden Ansprüche, **dadurch gekennzeichnet, dass** Schritt d während mindestens 5 min, vorzugsweise mindestens 15 min, noch bevorzugter mindestens 30 min durchgeführt wird,

6. Verfahren nach einem der vorhergehenden Ansprüche, **dadurch gekennzeichnet, dass** Schritt c die Folgenden Schritte umfasst:
c1. Vorerhitzen und Trocknen des benutzten saugfähigen Artikels bei einer ersten Temperatur währen eines ersten Zeitraums,
c2. Erhitzen und Trocknen des benutzten saugfähigen Artikels bei einer zweiten Temperatur während eines zweiten Zeitraums,
wobei die erste Temperatur niedriger als die zweite Temperatur ist und/oder der erste Zeitraum kürzer als der zweite Zeitraum ist.

7. Verfahren nach einem der vorhergehenden Ansprüche, **dadurch gekennzeichnet, dass** Schritt d auf die Ausführung von Schritt c in weniger als 40 min folgt.

8. Verfahren nach einem der vorhergehenden Ansprüche, **dadurch gekennzeichnet, dass** die Ausführungszeit von Schritt d kürzer als die Ausführungszeit von Schritt c ist, vorzugsweise die Ausführungszeit von Schritt d ungefähr die Ausführungszeit von Schritt c ist.

9. Verfahren nach einem der vorhergehenden Ansprüche, **dadurch gekennzeichnet, dass** Schritt b durch eine Anwendung von physischen Kräften durchgeführt wird, wie z. B. Schreddern und/oder Schneiden und/oder Zermahlen und/oder Schütteln.

10. Verfahren nach einem der vorhergehenden Ansprüche, **dadurch gekennzeichnet, dass** alle Schritte bei einem Druck von mindestens 85 kPa bis höchstens 115 kPa, vorzugsweise 90 kPa bis höchstens 110 kPa, noch bevorzugter von mindestens 95 kPa bis höchstens 105 kPa durchgeführt werden.

11. Verfahren nach einem der vorhergehenden Ansprüche, **dadurch gekennzeichnet, dass** nach Ausführung von Schritt a - d das Verfahren den zusätzlichen Schritt e des Trennens der kleineren Teile gemäß ihrer chemischen und/oder physischen Struktur zum Recycling umfasst.

12. Verfahren nach einem der vorhergehenden Ansprüche, **dadurch gekennzeichnet, dass** die Umgebungsbedingungen von Schritt b und/oder Schritt c frei von wässriger Lösung sind

13. Verfahren nach einem der vorhergehenden Ansprüche, **dadurch gekennzeichnet, dass** die saugfähigen Artikel benutzt sind und mindestens teilweise mit Urin kontaminiert sind.

14. Verfahren nach einem der vorhergehenden Ansprüche, **dadurch gekennzeichnet, dass** die benutzten oder unbenutzten saugfähigen Artikel mit potenziell pathogenen mikrobiellen Stämmen kontaminiert sind, ausgewählt aus der Gruppe von *Escherichia Coli, Enterococcus Faecalis, Staphylococcus Aureus, Salmonella Enterica* und *Candida Albicans.*

## Revendications

1. Procédé de stérilisation d'un ou plusieurs articles absorbants usagés ou non usagés comprenant dans l'ordre les étapes de :
a. fourniture des articles absorbants usagés ou non usagés,
b. séparation desdits articles absorbants en parties plus petites,
c. chauffage et séchage au moins partiel desdits articles absorbants,
d. application directe d'ozone à l'état gazeux sur lesdits articles absorbants,
**caractérisé en ce que** le chauffage de l'étape c est effectué à une température d'au moins 80° C à au plus 200° C et **en ce que** l'ozone à l'état gazeux a une concentration d'au moins 960 ppm ou 2,05567 g/m³ dans l'air à au plus 3000 ppm ou 6,42398 g/m³ dans l'air,

2. Procédé selon la revendication 1, **caractérisé en ce que** l'ozone à l'état gazeux a une concentration d'au moins 1440 ppm ou 3,08351 g/m³ dans l'air à au plus 2800 ppm ou 5,99572 g/m³ dans l'air, a préférablement une concentration d'au moins 1920 ppm ou 4,11135 g/m³ dans l'air à au plus 2600 ppm ou 5,56745 g/m³ dans l'air, a, plus préférablement encore, une concentration d'environ 2400 ppm ou 5,13919 g/m³ dans l'air.

3. Procédé selon l'une quelconque des revendications précédentes, **caractérisé en ce que** l'étape c est effectuée pendant au moins 5 min jusqu'à au plus 120 min, préférablement pendant au moins 30 min jusqu'à au plus 90 min, plus préférablement pendant au moins 45 min jusqu'à au plus 75 min, plus préférablement encore pendant environ 60 min.

4. Procédé selon l'une quelconque des revendications précédentes, **caractérisé en ce que** le chauffage de l'étape c est effectué à une température d'au moins 80° C à au plus 110° C, préférablement d'au moins 80° C à au plus 95° C, encore plus préférablement à une température d'au moins 80° C à au plus 90° C, encore plus préférablement à une température d'environ 85° C.

5. Procédé selon l'une quelconque des revendications précédentes, **caractérisé en ce que** l'étape d est effectuée pendant au moins 5 min, préférablement au moins 15 min, encore plus préférablement au moins 30 min.

6. Procédé selon l'une quelconque des revendications précédentes, **caractérisé en ce que** l'étape c comprend les étapes de :
c1. préchauffage et séchage de l'article absorbant usagé à une première température pendant une première quantité de temps,
c2. chauffage et séchage de l'article absorbant usagé à une seconde température pendant une seconde quantité de temps,
dans lequel ladite première température est inférieure à ladite seconde température et/ou ladite première quantité de temps est inférieure à ladite seconde quantité de temps.

7. Procédé selon l'une quelconque des revendications précédentes, **caractérisé en ce que** l'étape d suit la réalisation de l'étape c en moins de 40 min.

8. Procédé selon l'une quelconque des revendications précédentes, **caractérisé en ce que** le temps de réalisation de l'étape d est inférieur au temps de réalisation de l'étape c, plus préférablement le temps de réalisation de l'étape d est environ égal au temps de réalisation de l'étape c.

9. Procédé selon l'une quelconque des revendications précédentes, **caractérisé en ce que** l'étape b est effectuée par une application de forces physiques telles que déchiquetage et/ou découpage et/ou écrasement et/ou agitation.

10. Procédé selon l'une quelconque des revendications précédentes, **caractérisé en ce que** toutes les étapes sont effectuées à une pression d'au moins 85 kPa à au plus 115 kPa, préférablement de 90 kPa à au plus 110 kPa, encore plus préférablement d'au moins 95 kPa à au plus 105 kPa.

11. Procédé selon l'une quelconque des revendications précédentes, **caractérisé en ce qu'**après la réalisation des étapes a à d, le procédé comprend l'étape e supplémentaire de séparation des parties plus petites selon leur structure chimique et/ou physique pour recyclage.

12. Procédé selon l'une quelconque des revendications précédentes, **caractérisé en ce que** les conditions environnementales de l'étape b et/ou de l'étape c sont exemptes de solution aqueuse.

13. Procédé selon l'une quelconque des revendications précédentes, **caractérisé en ce que** les articles absorbants sont usagés et au moins partiellement contaminés par de l'urine.

14. Procédé selon l'une quelconque des revendications précédentes, **caractérisé en ce que** les articles absorbants usagés ou non usagés sont contaminés par des souches microbiennes potentiellement pathogènes sélectionnées dans le groupe des Escherichia Coli, Enterococcus Faecalis, Staphylococcus Aureus, Salmonella Enterica et Candida Albicans.
